# EUROPEAN PATENT SPECIFICATION

(11) **EP 3 616 415 B1**
(45) Date of publication and mention of the grant of the patent: **19.07.2023**
(21) Application number: 18790002.2
(22) Date of filing: 23.04.2018
(51) Int. Cl.: H04R 25/00, A61N 1/08, A61N 1/37, A61N 1/372

(54) **MRI-SAFETY AND FORCE OPTIMIZED IMPLANT MAGNET SYSTEM**
MRI-SICHERHEITS- UND KRAFTOPTIMIERTES IMPLANTATMAGNETSYSTEM
SYSTÈME D'AIMANT D'IMPLANT À SÉCURITÉ IRM ET À FORCE OPTIMISÉES

(30) Priority: 24.04.2017 US 201762488932 P
(43) Date of publication of application: 04.03.2020
(73) Proprietor: Med-El Elektromedizinische Geraete GmbH, 6020 Innsbruck (AT)
(72) Inventor: EIGENTLER, Thomas Wilhelm, A-6073 Sistrans (AT)
(74) Representative: Lucke, Andreas
(86) International application number: PCT/US2018/028785
(87) International publication number: WO 2018/200347

(56) References cited:
- WO-A1-2016/190886
- WO-A1-2016/199096
- WO-A1-2017/044523
- US-A1- 2002 019 669
- US-A1- 2004 012 470
- US-A1- 2011 264 172
- US-A1- 2012 219 166
- US-A1- 2014 107 400
- US-A1- 2014 321 681
- US-A1- 2016 361 537

## Description

This application claims priority from U.S. Provisional Patent Application 62/488,932, filed April 24, 2017.

### FIELD OF THE INVENTION

The present invention relates to implantable hearing devices such as cochlear implants, and specifically, to implantable magnets in such devices.

### BACKGROUND ART

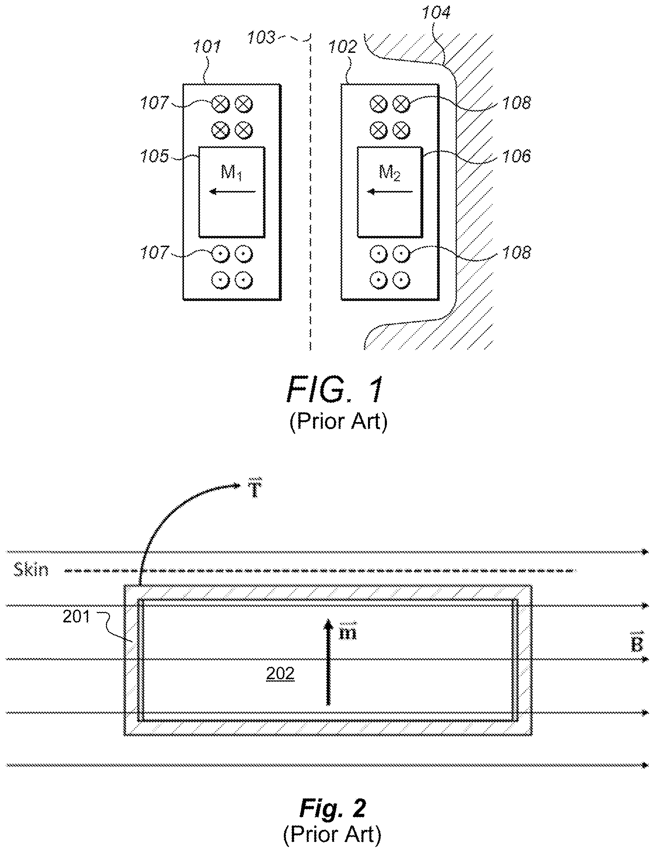

Some hearing implants such as Middle Ear Implants (MEI's) and Cochlear Implants (CI's) employ cooperating attachment magnets located in the implant and the external part to hold the external part in place over the implant. For example, as shown in Figure 1, a typical hearing implant system may include an external transmitter housing **101** containing transmitting coils **107** and an external attachment magnet **105.** The external attachment magnet **105** has a conventional cylindrical disc-shape and a north-south magnetic dipole having an axis that is perpendicular to the skin of the patient as shown. Implanted under the patient's skin is a corresponding receiver assembly **102** having similar receiving coils **108** and an implant magnet **106.** The implant magnet **106** also has a cylindrical disc-shape and a north-south magnetic dipole having a magnetic axis that is perpendicular to the skin of the patient as shown. The internal receiver housing **102** is surgically implanted and fixed in place within the patient's body. The external transmitter housing **101** is placed in proper position over the skin covering the internal receiver assembly **102** and held in place by interaction between the magnets **105** and **106** thus, the internal magnetic field lines and the external magnetic field lines. Rf signals from the transmitter coils **107** couple data and/or power to the receiving coil **108** which is in communication with an implanted processor module (not shown).

One problem with the typical hearing implant, as shown in Figure 1, arises when the patient undergoes Magnetic Resonance Imaging (MRI) examination. Interactions occur between the implant magnet and the applied external magnetic field for the MRI. As shown in Figure 2, the direction of the magnetic dipole **m̅** of the implant magnet **202** is essentially perpendicular to the skin of the patient. In this example, the strong static magnetic field **B̅** from the MRI creates a torque **T̅** = **m̅ × B̅** on the internal magnet **202,** which may displace the internal magnet **202** or the whole implant housing **201** out of proper position. Among other things, this may damage the implant or the adjacent tissue of the patient. In addition, the external magnetic field **B̅** from the MRI may reduce, remove or invert the magnetic dipole **m̅** of the implant magnet **202** so that it may no longer be able or strong enough to hold the external transmitter housing in proper position. Torque and forces acting on the implant magnet and demagnetization of the implant magnet is especially an issue with MRI field strengths exceeding 1.5 Tesla.

Thus, for existing implant systems with magnet arrangements, it is common to either not permit MRI, or at most limit use of MRI to lower field strengths. Other existing solutions include use of a surgically removable magnets, spherical implant magnets (e.g. U.S. Patent 7,566,296), and various ring magnet designs (e.g., U.S. Patent Publication 2011/0022120).

U.S. Patent 8,634,909 describes an implant magnet having a diametrical magnetization, where the magnetic axis is parallel to the end surfaces of a disc shaped implant magnet-that is, perpendicular to the conventional magnetic axis of a disc-shaped implant magnet. The magnet is then held in a receptacle that allows the magnet to rotate about its center axis in response to an external magnetic field such as from an MRI to realign and avoid creating torque. But this rotation is only possible around a single axis, the central axis.

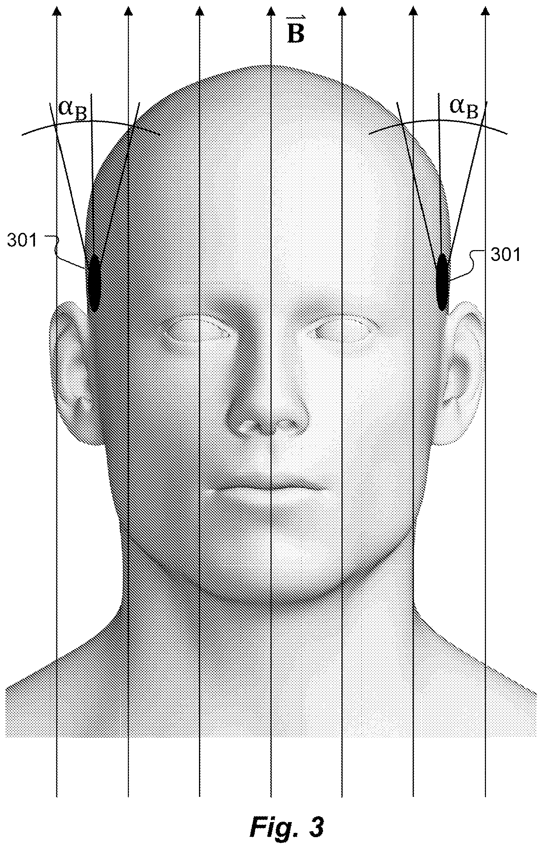

Figure 3 shows the head of a patient with bilateral hearing implants 301 having such an implant magnet in the presence of a typical MRI scanning magnetic field Bₒ, which is aligned along the longitudinal axis of the patient. The magnetization axis of the hearing implants **301** is angled with respect to the magnetic field **B̅** at some relative angle α_{B} as shown in Figure 3, which can create an undesirable torque on the hearing implants **301.** This relative angle α_{B} is dependent on the individual patient's anatomy and the exact implant position, for example on the skull of the patient.

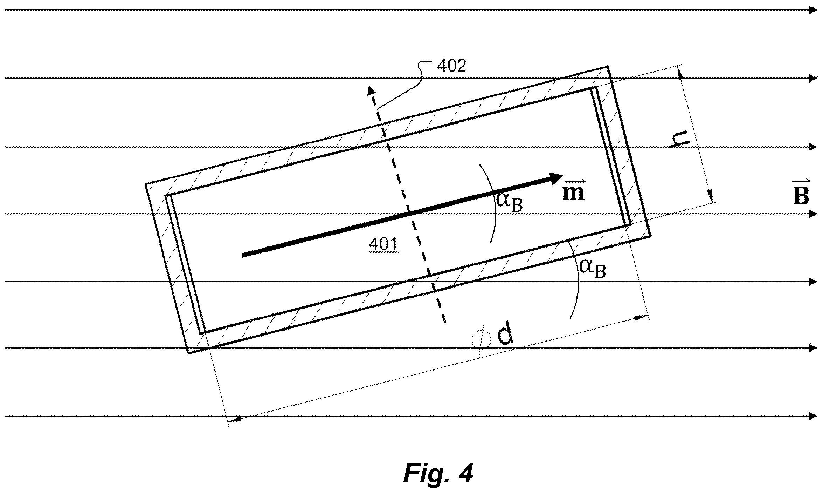

Figure 4 shows in greater detail the geometry of an implant magnet **401** with a magnetic dipole **m̅** that is parallel to the skin, and an MRI scanning magnetic field **B̅** aligned along the longitudinal symmetry axis. The cylindrical disc shape of the implant magnet **401** has a height h and a diameter Ød. Depending on the specific orientation of the implant within the patient, there will be a relative angle α_{B} between the direction of the magnetic dipole **m̅** of the implant magnet **401** and the static magnetic field **B̅**. The relative angle α_{B} also remains when implant magnet **401** is rotatable about its cylindrical axis **402**, as for example described in U.S. Patent 8,634,909. This relative angle α_{B} leads to a torque force on the implant magnet **401**, where the torque **T̅** = **m̅ × B̅** , and the force at the circumference of the stiff structure is **F̅** = **T̅** / **D**, where D is the distance or diameter of the stiff structure surrounding the implant magnet **401**.

A WO 2016/199096 A1 discloses an implantable medical device, including a magnet and a body encompassing the magnet, wherein the implantable medical device includes structural components in the body configured to move away from one another upon initial rotation of the magnet relative to the body when the magnet is subjected to an externally generated magnetic field, thereby limiting rotation of the magnet beyond the initial rotation.

US 2014/0321681 A1 discloses an implantable floating mass transducer for a hearing implant system in an implant patient. A cylindrical transducer housing contains a cylindrical inner mass magnet having an inner magnetic field with a first field direction. One or more signal drive coils are on the outer housing surface for conducting a transducer drive signal current to produce a signal magnetic field that interacts with the inner magnetic field to create vibration of the inner mass magnet which is coupled by the transducer housing to the internal hearing structure for sound perception by the implant patient. A ring-shaped outer offset magnet is positioned around the outer housing surface with an outer magnetic field having a second field direction opposite to the first field direction so as to offset the inner magnetic field to minimize their combined magnetic field and thereby minimize magnetic interaction of the transducer with any external magnetic field.

US 2012/0219166 A1 discloses a floating mass transducer for a hearing implant, including a cylindrical transducer housing that is attachable to a middle ear hearing structure and that has an outer surface with one or more electric drive coils thereon. A cylindrical transducer magnet arrangement is positioned within an interior volume of the transducer housing and includes a magnetic pair of an inner rod magnet and an outer annular magnet. Current flow through the drive coils creates a magnetic field that interacts with the magnetic fields of the transducer magnet arrangement to create vibration in the transducer magnet which is coupled by the transducer housing to the middle ear hearing structure for perception of sound. Opposing magnetic fields of the transducer magnet arrangement cancel each other to minimize their combined magnetic field and thereby minimize magnetic interaction of the transducer magnet arrangement with any external magnetic field.

WO 2017/044523 A1 discloses a magnet arrangement for an implantable medical device. An implantable coil case contains a communications coil and is made of biocompatible resilient material with a top lateral surface and an opposing bottom medial surface. A magnet receptacle is located within the coil case and has opposing receptacle openings in the top lateral surface and the bottom medial surface. An implant magnet fits within the magnet receptacle and has opposing end surfaces, and a center body region located between the end surfaces. The center body diameter is larger than the end diameters. The implant magnet and the magnet receptacle are configured to cooperate to permit the implant magnet to be inserted into or removed from the magnet receptacle through either of the receptacle openings.

### SUMMARY OF THE INVENTION

In accordance with the present invention, there is provided a magnet arrangement for an implantable hearing device as set out in claim 1.

An implantable magnet has a modified disc shape with a primary center rotation axis, a cylindrical height, a diameter, an outer circumference and opposing end surfaces. The implant magnet shape has at least one cross section view in which the primary center rotating axis is defined where the height of the magnet system is greatest and an axis normal to the cross section view is defining the secondary deflection axis. This magnet shape is capable of responding to an external magnetic field by rotating about the primary center rotation axis. The implant magnet shape has at least one cross-sectional view in which the cylindrical diameter corresponds to a horizontal coordinate axis, the primary center rotation axis corresponds to a vertical coordinate axis, and the height between the end surfaces is greatest. The height between the end surfaces progressively decreases from the primary center rotation axis along the cylindrical diameter towards the outer circumference to define a secondary deflection angle with respect to the horizontal coordinate axis so that the implant magnet is capable of responding to the external magnetic field by deflecting within the secondary deflection angle about a secondary deflection axis defined by a cylinder diameter normal to the at least one cross-sectional view.

The magnet arrangement further comprises a magnet housing enclosing a cylindrical shaped interior volume that contains the implant magnet. The implant magnet then is configured to securely fit within the interior volume so as to allow free alignment to an external magnetic field about the primary rotating axis as is limited partial rotation about the secondary deflection axis. In such embodiments, the interior volume may contain a damper oil which surrounds the implant magnet and/or at least one ferromagnetic domain which enabled a magnetic fixation of the implant magnet inside the embodiment. The implant magnet may include one or more low-friction contact surfaces configured to connect the implant magnet to the magnet housing.

The at least one cross-sectional view may be exactly one cross-sectional view, or it may be every cross-sectional view in which the cylindrical diameter corresponds to a horizontal coordinate axis and the primary center rotation axis corresponds to a vertical coordinate axis.

Embodiments of the present invention also include a hearing implant system containing a magnet arrangement according to any of the foregoing.

### BRIEF DESCRIPTION OF THE DRAWINGS

Figure 1 shows portions of a typical cochlear implant system and the magnetic interaction between the implant magnet and the external holding magnet.
Figure 2 illustrates the force interactions that can occur between an implant magnet and the applied external magnetic field for an MRI system.
Figure 3 the head of a patient with bilateral cochlear implants in the presence of a typical MRI scanning magnetic field.
Figure 4 shows geometry of an implant magnet with a magnetic dipole parallel to the skin and an MRI scanning magnetic field.
Figure 5 shows cross-sectional view geometry of a modified disc-shaped implant magnet according to an embodiment of the present invention.
Figure 6 shows a cross-sectional view of an implant magnet enclosed within a magnet housing.
Figure 7 shows geometry of an implant magnet arrangement according to Figure 6 in an MRI scanning magnetic field.
Figures 8A-8B show elevated perspective views of a rotationally symmetric and a non-rotationally symmetric implant magnet according to embodiments of the present invention.
Figure 9 shows a cross-sectional view of an implant magnet arrangement with friction-reducing surfaces according to an embodiment of the present invention.

### DETAILED DESCRIPTION OF SPECIFIC EMBODIMENTS

Embodiments of the present invention are directed to an improved implant magnet that can achieve a lower mechanical force during an MRI for a given magnetization or magnet strength. The inventive implant magnet has a limited deflection rotation about a secondary deflection axis to reduce the torque created by the static magnetic field **B̅** in the MRI-scanner. This, in turn, allows use of a stronger implant magnet with the same mechanical torque during MRI.

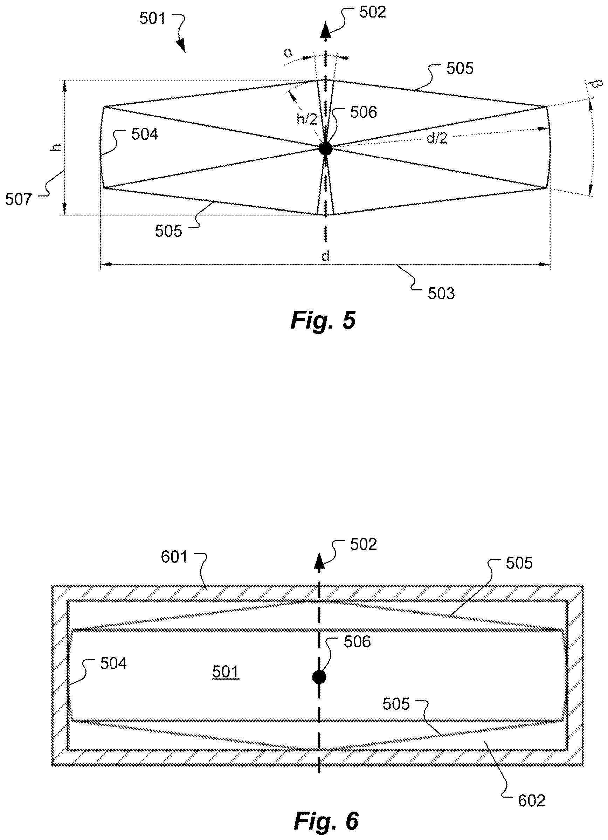

Figure 5 shows the cross-sectional view geometry of an implant magnet **501** according to one embodiment of the present invention, with a center rotation axis **502,** a cylindrical height **507** and diameter **503,** an outer circumference **504,** and opposing end surfaces **505.** The implant magnet **501** is capable of responding to an external magnetic field **B̅** by rotating about the center rotation axis **502.** And the shape of the implant magnet **501** has at least one cross-sectional view as shown in Figure 5 where the cylindrical diameter **503** corresponds to a horizontal coordinate axis, the primary center rotation axis **502** corresponds to a vertical coordinate axis. The height **507** of the implant magnet **501** between the end surfaces **505** is greatest at the primary center rotation axis **502** and progressively decreases from the primary center rotation axis **502** along the cylindrical diameter **503** towards the outer circumference **504.**

Figure 6 shows a cross-sectional view of a further specific embodiment with a magnet housing **601** that encloses a cylindrical shaped interior volume **602** that contains the implant magnet **501.** The implant magnet **501** is configured to securely fit within the interior volume **602** so as to be freely rotatable about the primary center rotation axis **502** and the secondary deflection axis **506.** In such embodiments, the interior volume **602** may contain a damper oil (to reduce rattler noise) which surrounds the implant magnet **501.**

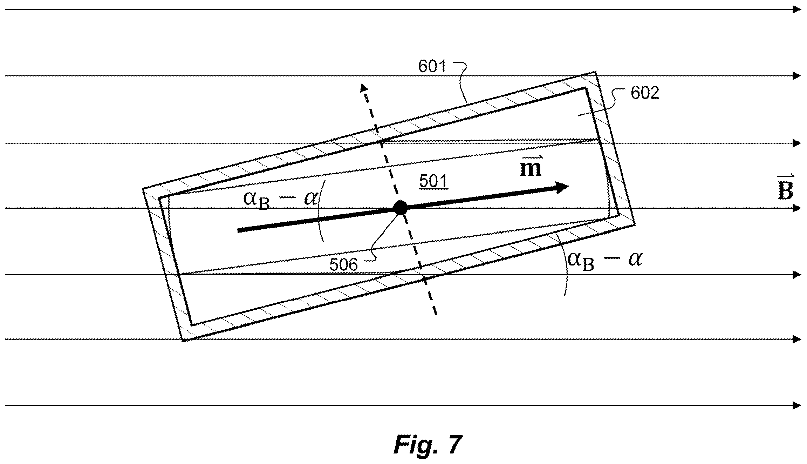

The geometry of the implant magnet **501** defines a secondary deflection angle α_{B} with respect to the horizontal coordinate axis so that the implant magnet **501** is capable of responding to the external magnetic field **B̅** , as shown in Figure 7, by deflecting within the secondary deflection angle α_{B} about a secondary deflection axis **506** which is normal to the at least one cross-sectional view, up until further secondary rotation is prevented by the end surfaces **505** pressing against the inner surface of the magnet housing **601** as shown in Figure 7.

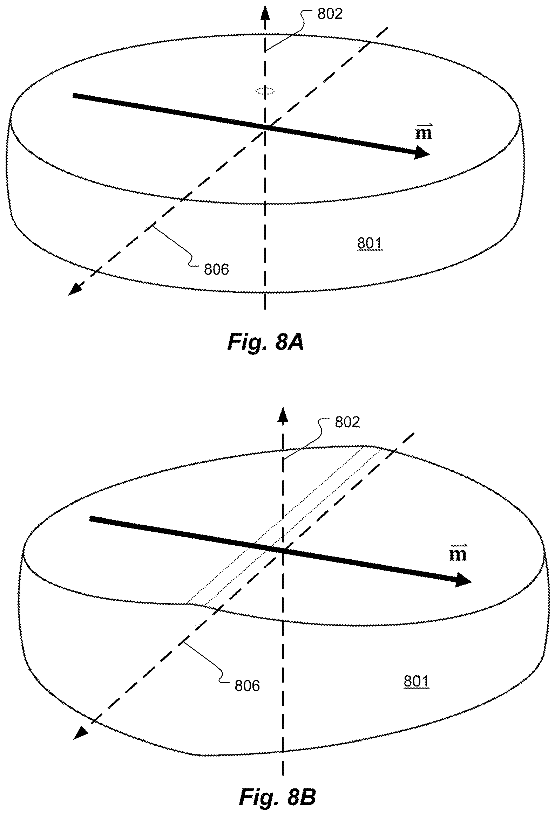

Figures 8A-8B show elevated perspective views of two different shape approaches to an implant magnet **801** according to an embodiment of the present invention. The implant magnet **801** shown in Figure 8A is rotationally symmetric. The end surfaces on the top and bottom of the disc-shaped implant magnet **801** form two rounded cones centered around the primary center rotation axis **802** with a chamfer radius of half the magnet height. Every cross-sectional view through the end surfaces will be such that the height is greatest at the center of the primary center rotation axis **802** and progressively decreases radially outward towards the outer circumference. To enable a secondary deflection around a secondary deflection axis **806,** the edges of the cylindrical diameter are chamfered with the radius of the half diameter. In such a rotationally symmetric implant magnet **801** the diametrical magnetization in every direction is normal to the primary rotation axis **802.**

The implant magnet **801** shown in Figure 8B is non-rotationally symmetric design with a rounded dam-shaped design on the top and bottom of the cylindrical implant magnet **801** with the radius of the chamfers the same as in the symmetric design in Figure 8A. For such a non-rotationally symmetric shape, the direction of the magnetic dipole **m̅** has to align normal to the secondary deflection axis **806,** which is in turn parallel to the top and bottom line of the dam-shape. It will be appreciated in this embodiment, there is just a single cross-sectional view where the magnet height is greatest at the primary center rotation axis **802** and progressively decreases radially outward towards the outer circumference.

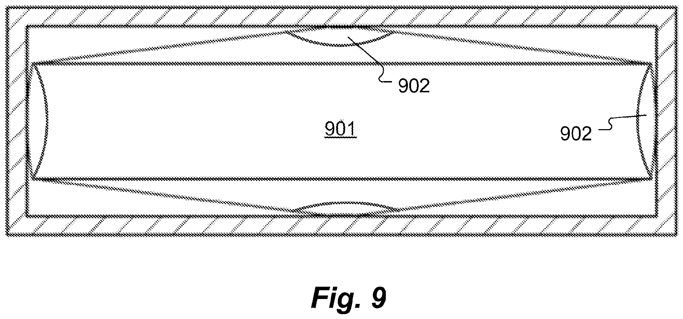

Figure 9 shows a cross-sectional view of a further specific embodiment where the implant magnet **901** includes one or more low-friction contact surfaces **902**, e.g. made of titanium, that are configured to connect the implant magnet **901** to the magnet housing; for example, at the center axis of symmetry and/or at the outer circumference.

Although various exemplary embodiments of the invention have been disclosed, it should be apparent to those skilled in the art that various changes and modifications can be made which will achieve the advantages of the invention without departing from the scope of the appended claims.

## Claims

1. A magnet arrangement for an implantable hearing device; the arrangement comprising:
an implant magnet (501, 801, 901), which is an implantable holding magnet, having a modified disc shape with a primary center rotation axis (502, 802), a cylindrical height (507) and diameter (503), an outer circumference (504), and opposing end surfaces (505);
wherein the implant magnet (501, 801, 901) is capable of responding to an external magnetic field by rotating about the primary center rotation axis (502, 802), and
wherein the implant magnet (501, 801, 901) shape has at least one cross-sectional view in which:
i. the cylindrical diameter (503) corresponds to a horizontal coordinate axis,
ii. the primary center rotation axis (502, 802) corresponds to a vertical coordinate axis,
iii. height (507) between the end surfaces (505) is greatest at the primary center rotation axis (502, 802), and
iv. height (507) between the end surfaces (505) progressively decreases from the primary center rotation axis (502, 802) along the cylindrical diameter (503) towards the outer circumference (504) to define a secondary deflection angle with respect to the horizontal coordinate axis so that the implant magnet (501, 801, 901) is capable of responding to the external magnetic field by deflecting within the secondary deflection angle about a secondary deflection axis (506, 806) defined by a cylinder diameter normal to the at least one cross-sectional view,
wherein the magnet arrangement further comprises:
a magnet housing (601) enclosing a cylindrical shaped interior volume (602) containing the implant magnet (501, 801, 901), wherein the implant magnet (501, 801, 901) is configured to securely fit within the interior volume (602) so as to be freely rotatable about the primary center rotating axis and about the secondary deflection axis (506, 806).

2. The magnet arrangement according to claim 1, wherein the interior volume (602) contains a damper oil which surrounds the implant magnet (501, 801, 901).

3. The magnet arrangement according to claim 1, wherein the interior volume (602) contains at least one ferromagnetic domain which surrounds the implant magnet (501, 801, 901).

4. The magnet arrangement according to claim 1, wherein the implant magnet (501, 801, 901) includes one or more low-friction contact surfaces configured to connect the implant magnet (501, 801, 901) to the magnet housing (601).

5. The magnet arrangement according to claim 4, wherein the one or more contact surfaces are located at the center axis of symmetry.

6. The magnet arrangement according to claim 4, wherein the one or more contact surfaces are located at the outer circumference (504).

7. The magnet arrangement according to claim 1, wherein the implant magnet (801) is of a non-rotationally symmetric design with respect to the primary center rotation axis (802).

8. The magnet arrangement according to claim 1, wherein the at least one cross-sectional view is every cross-sectional view in which the cylindrical diameter (503) corresponds to a horizontal coordinate axis and the primary center rotation axis (502, 802) corresponds to a vertical coordinate axis thus, a geometric rotationally symmetric design.

9. A hearing implant system containing a magnet arrangement according to any of claims 1-8.

## Patentansprüche

1. Magnetanordnung für eine implantierbare Hörvorrichtung, wobei die Anordnung umfasst:
einen Implantatmagneten (501, 801, 901), der ein implantierbarer Haltemagnet ist, der eine modifizierte Scheibenform mit einer primären zentralen Drehachse (502, 802), einer zylindrischen Höhe (507) und einem zylindrischen Durchmesser (503), einem Außenumfang (504) und gegenüberliegenden Endflächen (505) aufweist;
wobei der Implantatmagnet (501, 801, 901) in der Lage ist, auf ein externes Magnetfeld durch Drehen um die primäre zentrale Drehachse (502, 802) zu reagieren, und
wobei die Form des Implantatmagneten (501, 801, 901) mindestens eine Querschnittsansicht aufweist, in der:
i. der zylindrische Durchmesser (503) einer horizontalen Koordinatenachse entspricht,
ii. die primäre zentrale Drehachse (502, 802) einer vertikalen Koordinatenachse entspricht,
iii. die Höhe (507) zwischen den Endflächen (505) an der primären zentralen Drehachse (502, 802) am größten ist, und
iv. die Höhe (507) zwischen den Endflächen (505) von der primären zentralen Drehachse (502, 802) entlang des zylindrischen Durchmessers (503) in Richtung des Außenumfangs (504) progressiv abnimmt, um einen sekundären Auslenkungswinkel in Bezug auf die horizontale Koordinatenachse zu definieren, so dass der Implantatmagnet (501, 801, 901) in der Lage ist, auf das externe Magnetfeld durch Auslenken innerhalb des sekundären Auslenkungswinkels um eine sekundäre Auslenkungsachse (506, 806), die durch einen Zylinderdurchmesser senkrecht zu der mindestens einen Querschnittsansicht definiert ist, zu reagieren,
wobei die Magnetanordnung ferner umfasst:
ein Magnetgehäuse (601), das ein zylindrisch geformtes Innenvolumen (602) umschließt, das den Implantatmagneten (501, 801, 901) enthält, wobei der Implantatmagnet (501, 801, 901) dazu konfiguriert ist, sicher in das Innenvolumen (602) zu passen, um frei um die primäre zentrale Drehachse und um die sekundäre Auslenkungsachse (506, 806) drehbar zu sein.

2. Magnetanordnung nach Anspruch 1, wobei das Innenvolumen (602) ein Dämpferöl enthält, das den Implantatmagneten (501, 801, 901) umgibt.

3. Magnetanordnung nach Anspruch 1, wobei das Innenvolumen (602) mindestens eine ferromagnetische Domäne enthält, die den Implantatmagneten (501, 801, 901) umgibt.

4. Magnetanordnung nach Anspruch 1, wobei der Implantatmagnet (501, 801, 901) eine oder mehrere reibungsarme Kontaktflächen umfasst, die dazu konfiguriert sind, den Implantatmagneten (501, 801, 901) mit dem Magnetgehäuse (601) zu verbinden.

5. Magnetanordnung nach Anspruch 4, wobei sich die eine oder die mehreren Kontaktflächen an der zentralen Symmetrieachse befinden.

6. Magnetanordnung nach Anspruch 4, wobei sich die eine oder die mehreren Kontaktflächen am Außenumfang (504) befinden.

7. Magnetanordnung nach Anspruch 1, wobei der Implantatmagnet (801) einen nicht rotationssymmetrischen Aufbau in Bezug auf die primäre zentrale Drehachse (802) aufweist.

8. Magnetanordnung nach Anspruch 1, wobei die mindestens eine Querschnittsansicht jede Querschnittsansicht ist, in der der zylindrische Durchmesser (503) einer horizontalen Koordinatenachse entspricht und die primäre zentrale Drehachse (502, 802) einer vertikalen Koordinatenachse entspricht, also einem geometrischen rotationssymmetrischen Aufbau.

9. Hörimplantatsystem, das eine Magnetanordnung nach einem der Ansprüche 1-8 enthält.

## Revendications

1. Agencement d'aimant pour un dispositif auditif implantable ; l'agencement comprenant :
un aimant d'implant (501, 801, 901), qui est un aimant de maintien implantable, ayant une forme de disque modifiée avec un axe de rotation central primaire (502, 802), une hauteur (507) et un diamètre (503) cylindriques, une circonférence externe (504), et des surfaces d'extrémité opposées (505) ;
dans lequel l'aimant d'implant (501, 801, 901) est capable de répondre à un champ magnétique externe par rotation autour de l'axe de rotation central primaire (502, 802), et dans lequel la forme de l'aimant d'implant (501, 801, 901) a au moins une vue en coupe transversale dans laquelle :
i. le diamètre cylindrique (503) correspond à un axe de coordonnée horizontale,
ii. l'axe de rotation central primaire (502, 802) correspond à un axe de coordonnée verticale,
iii. la hauteur (507) entre les surfaces d'extrémité (505) est la plus élevée au niveau de l'axe de rotation central primaire (502, 802), et
iv. la hauteur (507) entre les surfaces d'extrémité (505) diminue progressivement depuis l'axe de rotation central primaire (502, 802) le long du diamètre cylindrique (503) vers la circonférence externe (504) pour définir un angle de déviation secondaire par rapport à l'axe de coordonnée horizontale de sorte que l'aimant d'implant (501, 801, 901) est capable de répondre au champ magnétique externe par déviation de l'angle de déviation secondaire autour d'un axe de déviation secondaire (506, 806) défini par un diamètre cylindrique normal par rapport à l'au moins une vue en coupe transversale,
l'agencement d'aimant comprenant en outre :
un boîtier d'aimant (601) entourant un volume intérieur de forme cylindrique (602) contenant l'aimant d'implant (501, 801, 901), dans lequel l'aimant d'implant (501, 801, 901) est configuré pour s'ajuster étroitement dans le volume intérieur (602) de façon à pouvoir tourner librement autour de l'axe de rotation central primaire et autour de l'axe de déviation secondaire (506, 806).

2. Agencement d'aimant selon la revendication 1, dans lequel le volume intérieur (602) contient une huile d'amortisseur qui entoure l'aimant d'implant (501, 801, 901).

3. Agencement d'aimant selon la revendication 1, dans lequel le volume intérieur (602) contient au moins un domaine ferromagnétique qui entoure l'aimant d'implant (501, 801, 901).

4. Agencement d'aimant selon la revendication 1, dans lequel l'aimant d'implant (501, 801, 901) comprend une ou plusieurs surfaces de contact à faible frottement configurées pour relier l'aimant d'implant (501, 801, 901) au boîtier d'aimant (601).

5. Agencement d'aimant selon la revendication 4, dans lequel les une ou plusieurs surfaces de contact sont situées au niveau de l'axe de symétrie central.

6. Agencement d'aimant selon la revendication 4, dans lequel les une ou plusieurs surfaces de contact sont situées au niveau de la circonférence externe (504).

7. Agencement d'aimant selon la revendication 1, dans lequel l'aimant d'implant (801) a une conception ne présentant pas une symétrie de rotation par rapport à l'axe de rotation central primaire (802).

8. Agencement d'aimant selon la revendication 1, dans lequel l'au moins une vue en coupe transversale est chaque vue en coupe transversale dans laquelle le diamètre cylindrique (503) correspond à un axe de coordonnée horizontale et l'axe de rotation central primaire (502, 802) correspond à un axe de coordonnée verticale, par conséquent, une conception géométrique présentant une symétrie de rotation.

9. Système d'implant auditif contenant un agencement d'aimant selon l'une quelconque des revendications 1 à 8.
